(19) Europäisches Patentamt
European Patent Office
Office européen des brevets

(11) **EP 2 208 087 B1**

(12) **EUROPÄISCHE PATENTSCHRIFT**

(45) Veröffentlichungstag und Bekanntmachung des Hinweises auf die Patenterteilung:
**16.01.2013 Patentblatt 2013/03**

(21) Anmeldenummer: 08843783.5

(22) Anmeldetag: **16.10.2008**

(51) Int Cl.:
**G01T 1/164** *(2006.01)*

(86) Internationale Anmeldenummer:
**PCT/DE2008/001699**

(87) Internationale Veröffentlichungsnummer:
**WO 2009/056094 (07.05.2009 Gazette 2009/19)**

(54) **VERFAHREN ZUR POSITRONEN-EMISSIONS-TOMOGRAPHIE SOWIE PET-SCANNER**

METHOD FOR POSITRON EMISSION TOMOGRAPHY AND PET SCANNER

PROCEDE DE TOMOGRAPHIE PAR EMISSION DE POSITRONS (PET) ET SCANNER PET

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **30.10.2007 DE 102007052035**

(43) Veröffentlichungstag der Anmeldung:
**21.07.2010 Patentblatt 2010/29**

(73) Patentinhaber: **Forschungszentrum Jülich GmbH 52425 Jülich (DE)**

(72) Erfinder: **SCHEINS, Jürgen, Johann 52457 Aldenhoven (DE)**

(56) Entgegenhaltungen:
**US-A1- 2005 151 084     US-B1- 6 804 325**

• **MARK F SMITH ET AL: "Analysis of Factors Affecting Positron Emission Mammography (PEM) Image Formation" IEEE TRANSACTIONS ON NUCLEAR SCIENCE, IEEE SERVICE CENTER, NEW YORK, NY, US, Bd. 50, Nr. 1, 1. Februar 2003 (2003-02-01), XP011077994**

EP 2 208 087 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Positronen-Emissions-Tomographie.

In der Positronen-Emissions-Tomographie (PET) werden Szintillationsdetektoren eingesetzt, welche die Photonen detektieren, die bei der Vernichtung von Elektron-Positron-Paaren in der Probe paarweise in entgegen gesetzten Richtungen emittiert werden.

Stand der Technik

Bei einer PET-Messung gilt es, die Vernichtung von Elektron-Positron-Paaren in der Probe zu detektieren sowie zu lokalisieren, auf welcher Signallinie das jeweilige Ereignis stattgefunden hat. Aus einer Vielzahl solcher Ereignisse lässt sich dann durch tomographische Rekonstruktion die innere Aktivitätsverteilung der Probe ermitteln.

Bei jedem Ereignis werden zwei Photonen gleichzeitig in entgegen gesetzte Richtungen abgestrahlt, wobei die Orientierung im Raum nicht vorhersagbar, d. h. isotrop erfolgt. Daher sind in einem PET-Scanner eine Vielzahl von Detektoren um die Probe angeordnet, damit jedes der beiden auf ein Ereignis zurückgehenden Photonen auf einen Detektor trifft. Registrieren zwei der Detektoren in der Regel Szintillationsrkristalle mit PMT's (Photomultiplier Tubes) oder APD's (Avalanche Photodioden) gleichzeitig ein Ereignis, was mit einer Koinzidenzschaltung überwacht wird, ist die Information gewonnen, dass das Ereignis auf einer Verbindungslinie zwischen den beiden Detektoren stattgefunden hat. Diese Linie wird als Signallinie oder als Line-of-Response (LOR) bezeichnet.

Bislang werden die gemessenen Koinzidenzen einzelner Kristallkombinationen einer bestimmten Signallinie zugeordnet. Typischerweise wird eine Signallinie durch die Verbindung jeweils eines ausgewählten, zentralen oder repräsentiven Punktes in den beiden beteiligten Kristallen festgelegt.

Nachteilig sind die aufgenommenen Daten mit Rauschen sowie häufig mit Artefakten belastet. Diese Störungen werden durch die tomographische Rekonstruktion, die auf die Lösung eines inversen Problems hinausläuft, massiv verstärkt und mindern die Qualität des letztendlich aus der Rekonstruktion erhaltenen Bildes erheblich.

Die US 6,804,325 B1 offenbart ein Verfahren, bei dem eine Signallinie bei der tomographischen Rekonstruktion einer Probe ausgewertet wird, wobei zu jedem Ereignis eine Mehrzahl von Signallinien bestimmt und bei der tomographischen Rekonstruktion der Probe angewendet werden.

Aufgabe und Lösung

Es ist daher die Aufgabe der Erfindung, ein Verfahren zur Positronen-Emissions-Tomographie sowie einen mit diesem Verfahren arbeitenden PET-Scanner bereitzustellen, die genauere tomographische Rekonstruktionen liefern, als dies nach dem Stand der Technik möglich ist.

Diese Aufgabe wird erfindungsgemäß gelöst durch ein Verfahren gemäß Hauptanspruch 1.

Gegenstand der Erfindung

Ausgehend vom Oberbegriff des Anspruch 1 wird die Aufgabe erfindungsgemäß gelöst durch die im kennzeichnenden Teil des Anspruchs 1 angegebenen Merkmale.

Es wurde erkannt, dass der tatsächliche Informationsgehalt einer detektierten Koinzidenz aufgrund des Messprozesses räumlich "verschmiert" und nicht durch eine einzelne Signallinie repräsentiert wird bzw. darstellbar ist: Registrieren zwei räumlich ausgedehnte Detektoren eine Koinzidenz, so kann das zugehörige Ereignis nicht eindeutig einer bestimmten einzelnen Signallinie (LOR) zugeordnet werden. Vielmehr beobachtet die Detektorkombination aufgrund ihrer endlichen Ausdehnung bzw. Auflösung ein ganzes Bündel von Signallinien bzw. LOR's im 3D-Raum, die daher sämtlich mögliche Kandidaten der tatsächlich verursachenden Signallinie darstellen. Zudem ist die Detektionswahrscheinlichkeit jeder Signallinie eines Bündels aufgrund geometrischer Effekte nicht notwendigerweise homogen. Welche dieser Signallinien im Bündel tatsächlich die Registrierung auslöste, bleibt unbekannt, d.h. alle LOR's im Bündel sind gemäß ihrer Detektionswahrscheinlichkeit mögliche Quelle des Signals. Dabei variiert die Struktur der Signallinien-Bündel je nach Größe, Position und Orientierung der beiden jeweils betrachteten Detektoren. Dementsprechend wird auch die wahre Information über den Ort des Ereignisses, die durch das gleichzeitige Ansprechen zweier gegebener Detektoren geliefert wird, durch ein solches Bündel von Signallinien zwischen den beiden Detektoren repräsentiert, wobei die einzelnen Signallinien in unterschiedlicher Gewichtung beitragen können.

Des weiteren kommt es aufgrund von Compton-Streuung der Photonen innerhalb der Kristalle sowie durch statistisch fluktuierende Signale sowohl des Szintillationslichtes als auch der nachgelagerten Elektronik zu zusätzlichen Messungenauigkeiten und zusätzlicher Verschmierung der Messinformation, d. h. benachbarte Kristalle des tatsächlichen Kristalls werden irrtümlich als Ursprung der Koinzidenz detektiert.

Diese Verschmierung der Messinformation im Raum der Signallinien wird nach bisherigem Stand der Technik nicht adäquat im Projektionsraum berücksichtigt. Dies ist zu unterscheiden zu einer Berücksichtigung bzw. Modellierung der Verschmierung im Rahmen eines Rekonstruktionsalgorithmus mit dem Ziel einer Auflösungsverbesserung der resultierenden Bilder. Hierbei handelt es sich um ein bekanntes und gängiges Verfahren. Im Gegensatz dazu bereitet das erfindungsgemäße Verfahren die verwendeten Projektionsdaten in verbesserter Weise auf, bevor ein Rekonstruktionsverfahren angewendet wird. Der methodische Schritt der Modellierung der Verschmierung bzw. Auflösung direkt im Rekonstruktionsverfahren ist von der Erfindung unabhängig und ihr nachgelagert. Der Stand der Technik geht von einem vereinfachten, idealisierten Detektionsmodell zur Interpolation bzw. Aufbereitung zu tomographisch verwertbaren Projektionsdaten aus.

Mit dem erfindungsgemäßen Verfahren können erstmals Detektorkristallgrößen und generelle Systemauflösung bei der Definition der Projektionsdaten für die Rekonstruktion berücksichtigt werden. Dadurch können nunmehr geglättete Projektionsdaten ohne Minderung der tatsächlichen Auflösung des Scanner-Systems für die Bildrekonstruktion zur Verfügung gestellt werden, welche zu Bildern mit verbessertem Signal-Rauschverhältnis und damit besserer Bildqualität führen.

Vorteilhafte Weiterbildungen der Erfindung sind in den Unteransprüchen angegeben.

Die Figuren zeigen schematische Darstellungen sowie Bildergebnisse, die aus Daten unter Verwendung des erfindungsgemäßen Verfahrens resultieren.

Es zeigt:

Fig. 1: Verschiedene Detektionsortkombinationen in Kristallen

Fig. 2: Eine Illustration einer typischen Detektortopologie

Fig. 3: Ein Projektionsdaten-Histogramm

Fig.4: Transaxiale Schicht eines rekonstruierten Bildes, welches aus erfindungsgemäß generierten Projektionsdaten berechnet wurde.

Fig.5: Transaxiale Schicht eines rekonstruierten Bildes, welches aus Projektionsdaten generiert wurde, die gemäß dem Stand der Technik aus Detektor-Rohdaten erhalten wurden.

Fig.6a), b): Graphische Darstellung für die eindeutige Charakterisierung der Lage einer Signallinie im dreidimensionalen Raum.

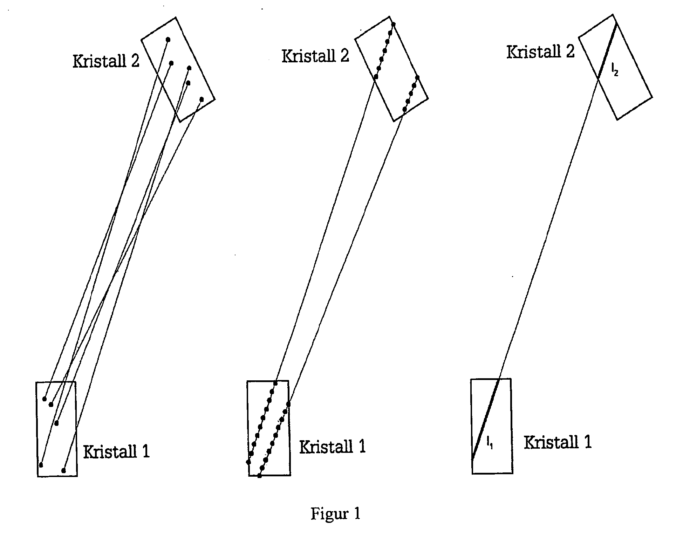

Figur 1 zeigt eine Abtastung einer Kristallkombination bestehend aus zwei verschiedenen Kristallen exemplarisch in einer 2D-Darstellung gemäß zweier Ausführungsformen des erfmdungsgemäßen Verfahrens.

Ausführungsform 1: (links) einzelne, jeweils zufällig bestimmte Punkte in beiden Kristallen definieren jeweils eine beobachtete Signallinie, (Mitte) verschiedene Kombinationen bestimmter Punkte bilden jeweils die gleiche Signallinie.

Ausführungsform 2: (rechts) die effektive Gewichtung einer Signallinie (LOR) ergibt sich aus dem Produkt $p_{12}=l_1*l_2$ der Schnittlängen dieser LOR mit den beteiligten Kristallen.

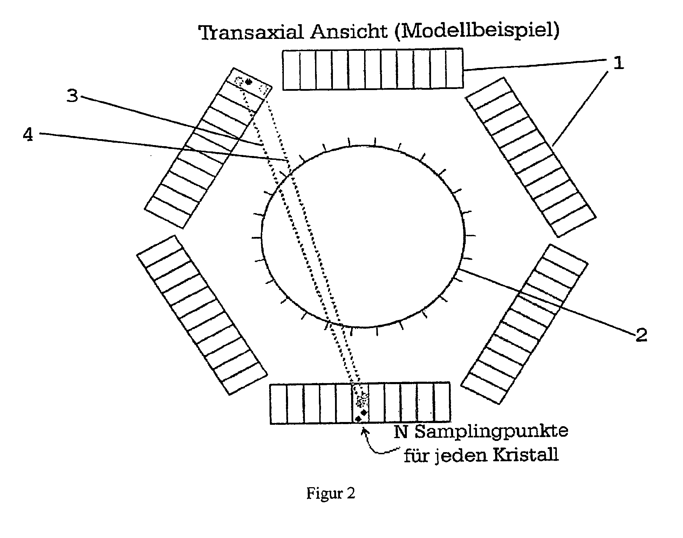

Figur 2 zeigt eine unregelmäßige Detektortopologie 1 (äußerer Teil) und einen Virtuellen Regulären Zylinderdetektor 2 (VRZ, innerer Teil) in einer transaxialen Ansicht. Der VRZ wird in der Ausgestaltung des erfindungsgemäßen Verfahrens verwendet, in der die Schnittpunkte jeder für diese Rekonstruktion in Betracht zu ziehenden Signallinien mit ihm ermittelt und bei der Rekonstruktion so behandelt werden, als hätten sich dort Detektoren befunden. Jede physikalische Signallinie 3,4 (LOR) schneidet den Zylinder in genau zwei eindeutigen Punkten. Der Zylinder ist dabei für die Zuordnung der Schnittpunkte in eine endliche Anzahl von Teilflächen (virtuelle Detektoren) durch Einführung von Zylinderringen und regelmäßigen Ringunterteilungen segmentiert. Jeder der Schnittpunkte der LOR mit dem Zylinder liegt auf einer eindeutig bestimmten Teilfläche, so dass die von den geschnittenen Teilflächen aufgespannte Tube-of-Response (TOR), die zuzuordnende LOR jedenfalls enthält. Somit ist jeder physikalischen Signallinie eine korrespondierende TOR auf eindeutige Weise zugeordnet.

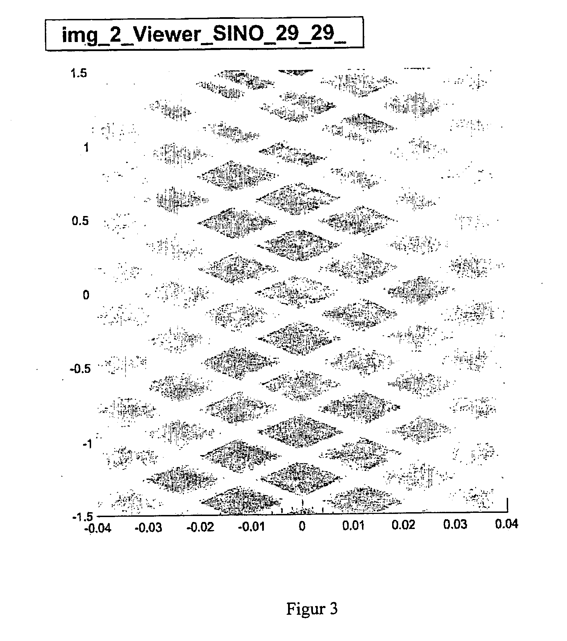

Figur 3 zeigt ein Projektionsdaten-Histogramm (Richtungswinkel auf der Y-Achse, radiale Koordinate auf der X-Achse) für einen bestimmten Zylinderring des VRZ-Modells; die gemessenen Koinzidenzdaten stammen von einem homogenen aktivitätsgefüllten Zylinder und wurden mit dem Clear PET Neuro PET Scanner (rotierende Detektoren) aufgenommen und mit einem vorberechneten Verteilungsschlüssel ins VRZ-Modell ohne weitere Korrekturen konvertiert. Die Sensitivitätslücken aufgrund von transaxialen Detektorlücken sind deutlich sichtbar. Mit zunehmender radialer Koordinate werden die geometrischen Informationen aufgrund des "Depth-of-Interaction"-Effekts verschmiert und entsprechen gemäß den zu Grunde liegenden geometrischen Eigenschaften einer Verschlechterung der Auflösung. Auflösungseinbußen durch "Depth-of-Interaction" für zunehmende radiale Abstände sind ursächlich durch die zunehmende Schrägstellung der Kristalle bezüglich der beteiligten LOR bedingt. Es handelt sich also ausschließlich um einen geometrischen Effekt. Durch die Schrägstellung erscheint die effektive Größe der jeweiligen Kristalle in radialer Richtung vergrößert, da die Kristalllängen zur Erhöhung der Sensitivität üblicherweise deutlich größer sind als die Breite bzw. Länge der Kristalloberflache (typisches Blockdesign). Dieser projizierte effektive Kristallquerschnitt mindert die radiale Ortsauflösung entsprechend. Durch das erfindungsgemäße Verfahren wird diese Verschmierung in den Projektionsdaten sichtbar.

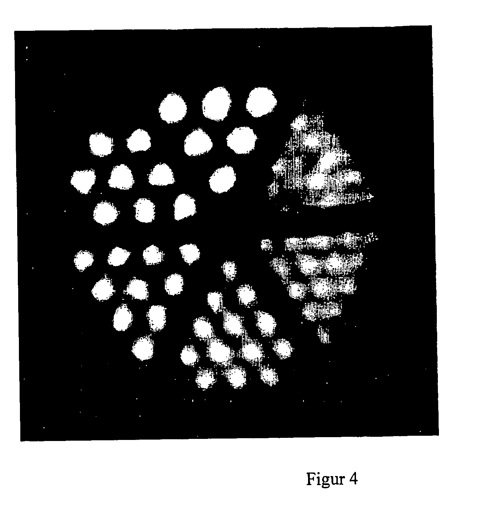

Figur 4 zeigt eine transaxiale Schicht eines rekonstruierten Bildes des Mini-Derenzo-Phantoms, gemessen mit dem Clear PET Neuro Scanner; die Messdaten wurden mit einer erfindungsgemäßen geometrischen Wahrscheinlichkeitsverteilung in das VRZ-Modell mit hoher Granularität konvertiert und anschließend im Rahmen des VRZ-Modells iterativ rekonstruiert. Die axial ausgerichteten, mit Aktivität befüllten Kapillaren haben Durchmesser von 2 mm (oben), 1,8 mm, 1,6 mm (links), 1,4 mm (unten), 1,2 mm und 1,0 mm (rechts). Die 1,2 mm Strukturen lassen sich noch deutlich differenzieren. Das Mini-Derenzo-Phantom hat insgesamt einen aktiven radialen Durchmesser von ca. 30 mm.

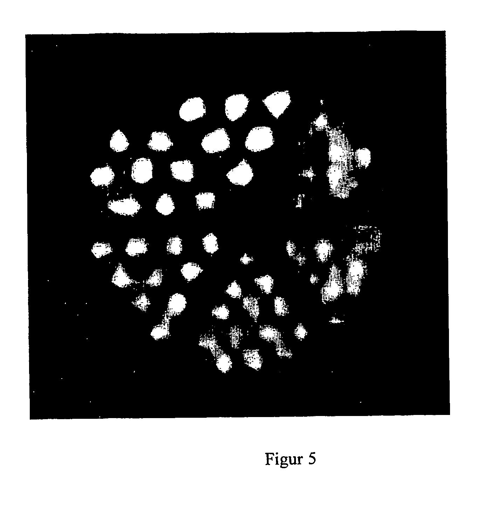

Figur 5 zeigt die gleiche transaxiale Schicht wie in Figur 4 mit identischen zu Grunde liegenden Messdaten wie in Fig. 4; die akquirierten Projektionsdaten wurden jedoch nicht mittels eines erfindungsgemäßen Verteilungsschlüssels konvertiert. Die iterative Rekonstruktion erfolgte mit einem Sinogramm basierten Programm [2] aus Projektionsdaten entsprechend dem Stand der Technik. Das Kontrast-Rausch-Verhältnis ist deutlich schlechter als in Figur 4. Strukturen unterhalb von 1,4 mm sind nicht mehr differenzierbar.

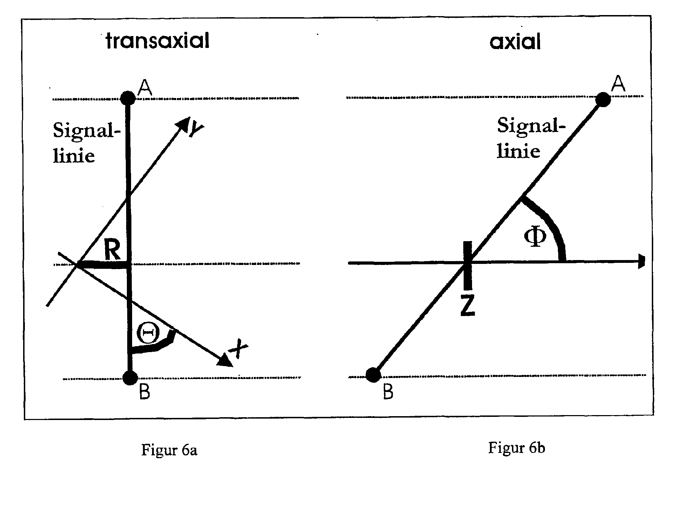

In den Figuren 6a und 6b werden die zur eindeutigen Charakterisierung der Lage der Signallinie notwendigen Parameter dargestellt, wie sie zur Definition von Projekrtionswerten gemäß Formel 1 verwendet werden. In den Figuren sind die Punkte A und B auf der Signallinie liegende Punkte. Figur 6a zeigt den in der transaxialen Ebene (xy-Ebene) liegenden Projektionswinkel $\Theta$ und die radiale Verschiebung R. Figur 6b zeigt die axiale Verschiebung Z entlang der axialen Richtung (Z-Achse) einer Detektorröhre und den Neigungswinkel $\Phi$ der Signallinie bezüglich der xy-Ebene.

Im Folgenden soll die Erfindung in ihrer allgemeinen Form beschrieben werden.

Erfindungsgemäß arbeitet das Positronen-Emissions-Tomographie-Verfahren mit folgenden Schritten:

a) durch ein Annihilationsereignis in der Probe werden zwei Photonen in entgegen gesetzten Richtungen emittiert,

b) von einer Vielzahl von um die Probe angeordneten Detektoren werden mindestens zwei durch die beiden Photonen zur Abgabe eines Signals veranlasst,

c) aus dem Ort der Detektoren, die ein Signal abgegeben haben, wird eine Signallinie bestimmt, auf der das Ereignis stattgefunden haben kann,

d) diese Signallinie wird bei der tomographischen Rekonstruktion der Probe ausgewertet, wobei zu jedem Ereignis eine Mehrzahl von Signallinien bestimmt und bei der tomographischen Rekonstruktion der Probe ausgewertet werden.

Hierdurch wird, wie eingangs beschrieben, das rekonstruierte Bild genauer und das Rauschen vermindert.

Eine Signallinie im Sinne der Erfindung ist eine Linie, die jeweils zwei verschiedene Punkte im dreidimensionalen Raum verbindet, wobei jeder der beiden Punkte jeweils einen lokalen Detektionsort eines Photons aus dem Annihilationsprozess beschreibt.

Jede beliebige Signallinie lässt sich eindeutig durch genau vier Parameter charakterisieren ($\Theta$, R, $\Phi$, Z) zwei in der transaxialen Ebene (XY-Ebene):

Projektionswinkel $\Theta$
Radiale Verschiebung R

sowie zwei in der axialen Richtung:

axiale Verschiebung Z (entlang Z-Koordinatenachse orthogonal zur XY-Ebene) Neigungswinkel $\Phi$ der Signallinie bezüglich der XY-Ebene

Sei S($\Theta$, R, $\Phi$, Z) das Signal einer Signallinie als Funktion der vier Parameter, so versteht man unter einem Projektionswert $P_i$ ein vierdimensionales Integral über ein spezifisches vierdimensionales Teilvolumen $V_i$ wie folgt:

$$P_i = \int\limits_{Vi} \iiint S(\Theta, R, \Phi, Z)\, d\Theta\, dR\, d\Phi\, dZ \qquad \text{Formel (1)}$$

Unter Projektionsdaten im Sinne der Erfindung versteht man dann eine Menge unterscheidbarer Projektionswerte $P_i$ resultierend aus der Integration gemäß Formel 1 mit verschiedenen spezifischen 4D-Teilvolumina $V_i$.

Unter einer Mehrzahl von Signallinien sind mindestens 2, vorzugsweise jedoch mehr, beispielsweise mindestens 10, 100, 1000, 100000 oder 1000000 zu verstehen.

Vorzugsweise wird jede mögliche Signallinie für jede Detektorkombination bei der Auswertung mit einer Wahrscheinlichkeitsverteilung gewichtet, die ein Maß dafür ist, auf welcher

Signallinie das Ereignis mit welcher Wahrscheinlichkeit stattgefunden hat. Die Gewichtung mit einer Wahrscheinlichkeitsfunktion hat zur Folge, dass die Projektionsdaten dem wahren physikalischen Informationsgehalt, der durch das Ereignis gegeben ist, besser entsprechen.

Die Wahrscheinlichkeitsverteilung kann in einer vorteilhaften Ausgestaltung der Erfindung durch eine Monte-Carlo-Technik ermittelt werden, indem eine Vielzahl von innerhalb der beiden Detektoren gelegenen Orten zufällig bestimmt wird und Signallinien zwischen einem Ort in dem einen Detektor und einem Ort in dem anderen Detektor als Signallinien gewertet werden, auf denen das Ereignis stattgefunden haben kann. In diesem Fall kann sie durch einen Verteilungsschlüssel angegeben bzw. zusammengefasst werden, gemäß Formel (2) beschrieben durch

$$(2) \quad S_{jk} = N_{jk} / N_{tot,j} \; .$$

Hierin ist:

$N_{tot,j}$: die Anzahl der generierten Signallinien aus jeweils einem zufällig ausgewählten Punkt in jedem Kristall für die Kristallkombination $j$

$k$ eine bestimmte getroffene TOR

$N_{jk}$ : die Anzahl der generierten LORs, die in der TOR $k$ liegen.

In einer weiteren Ausgestaltung der Erfindung wird die Wahrscheinlichkeitsverteilung ermittelt, indem für eine Vielzahl von Signallinien, die beide Detektoren schneiden, die Schnittlängen bestimmt werden, auf denen sie die Detektoren schneiden.

Dabei kann das Produkt aus den Längen für eine Signallinie als Maß für die Wahrscheinlichkeit gewertet werden, so dass das Ereignis auf dieser Linie stattgefunden hat. Bei dieser Verfahrensweise kann die Wahrscheinlichkeitsverteilung schneller ermittelt werden als durch zufällige Ermittlung einer Vielzahl von Orten innerhalb der beiden Detektoren.

Bei der Auswertung der Messinformation wird vorteilhaft die Ortsabhängigkeit der Sensitivität berücksichtigt. Dabei wird die Ortsabhängigkeit durch die Wahrscheinlichkeitsverteilung beschrieben. Durch die Berücksichtigung der Ortsabhängigkeit der Sensitivität der Detektorpaare können Projektionsdaten erzeugt werden, die dem wirklichen Informationsgehalt der detektierten Strahlung näher kommen, wodurch sich das Kontrast-Rausch-Verhältnis der rekonstruierten Bilder verbessert. Es können bei der Ermittlung der Projektionsdaten auch detektorspezifische Eigenschaften, wie beispielsweise Störungen, Inhomogenitäten oder Detektorlücken berücksichtigt werden. Detektorspezifische Verfälschungen der generierten Projektionsdaten werden minimiert oder ganz vermieden.

Für die Generierung von Wahrscheinlichkeitsverteilungen können auch Detektorsimulationen durchgeführt werden, welche beispielsweise die Compton-Streuung in den Kristallen nachbilden. Im typischen Blockdesign der Szintillationskristalle ergeben sich aufgrund der Compton-Streuung sowie messtechnisch bedingter Ungenauigkeiten Fehldetektionen von Kristallen, d. h. es kommt zu falschen LOR-Lokalisationen, welche eine über die Geometrie hinausgehende Verschlechterung der Ortsinformation bedingen. Diese zusätzlichen Informationsverluste lassen sich gleichfalls berücksichtigen. Diese Effekte sind jedoch komplexerer Art und lassen sich nur durch entsprechend aufwendige Simulationen oder Messungen quantifizieren.

Durch Monte Carlo-technische Simulation der physikalischen Prozesse in den Detektoren mittels Teilchenspur-Verfolgung im Detektormedium, z. B. Simulationsprogramme, wie sie in der Veröffentlichung von S. Agostinelli et al, "GEANT-A simulation toolkit", Nucl.Instr. Methods Phys. Res. A 506 (2003), p. 250-303 beschrieben werden, kann die Detektorauflösung bzw. Informationsverschmierung somit detailliert ermittelt werden. Die gewonnenen Informationen lassen sich verwenden, einen genaueren Verteilungsschlüssel zu ermitteln, der neben den Kristallgrößen auch das Übersprechverhalten der Signale auf benachbarte Kristalle beschreibt. Neben der Simulation können gezielte Messungen der Detektorantwort analog verwendet werden und Simulationen gegebenenfalls ersetzen.

In einer besonders vorteilhaften Ausgestaltung des erfindungsgemäßen Verfahrens werden vor der tomographischen Rekonstruktion die Schnittpunkte jeder für diese Rekonstruktion in Betracht zu ziehenden Signallinie (LOR) mit einer vorgegebenen geometrischen Form ermittelt und bei der Rekonstruktion so behandelt, als hätten sich dort Detektoren befunden. Hierbei wird die geometrische Form für die Ermittlung der Schnittpunkte vorzugsweise in eine endliche Anzahl von Teilflächen diskretisiert. Dabei wird weiterhin bevorzugt ein Kreis bzw. ein Zylinder als geometrische Form gewählt.

Es wurde erkannt, dass sich bei dieser Vorgehensweise die Symmetrie der geometrischen Form vorteilhaft zur Vereinfachung der tomographischen Rekonstruktion nutzen lässt. Je mehr Symmetrien die geometrische Form aufweist, desto geringer ist der nötige Aufwand der geometrischen Berechnung im Rahmen des Rekonstruktionsverfahrens. Der reduzierte Aufwand führt zu geringeren Rechenzeiten für die Bildrekonstruktion.

Diese Ausgestaltung des erfindungsgemäßen Verfahrens ermöglicht auch eine Trennung zwischen Detektorgeometriebeschreibung und optimierbaren Rekonstrruktionsalgorithmen für die Bildrekonstruktion: Der Rekonstruktionsalgorithmus braucht nur für eine einzige Geometrie, welche die für eine optimale Rechengeschwindigkeit vorteilhaften Symmetrien aufweist, optimiert zu werden. Wie auch immer der physisch vorhandene Scanner geometrisch beschaffen ist, die mit ihm aufgenommenen Daten können immer auf diese eine vorteilhafte Form projiziert werden, indem die Schnittpunkte der für diese Rekonstruktion in Betracht zu ziehenden Signallinie bzw. TORs mit dieser geometrischen Form bestimmt werden. Hierdurch können Bilder schneller rekonstruiert werden.

In Folgenden wird eine Vorrichtung, insbesondere ein Positronen-Emissions-Tomographie Scanner beschrieben, die zur Durchführung des erfindungsgemäßen Verfahrens geeignet ist und entweder über Mittel zur Bestimmung der Wahrscheinlichkeitsverteilung oder Mittel zur Bereitstellung der Wahrscheinlichkeit, dass ein von mindestens zwei Detektoren registriertes Ereignis auf einer gegebenen Signallinie zwischen den beiden Detektoren stattgefunden hat, verfügt.

Beispielsweise kann gemäß Alternative 2 ein Positronen-Emissions-Tomographie Scanner mit Datenträgern ausgestattet sein, auf dem die LOR-Wahrscheinlichkeitsverteilungen für Detektor-Paare in Form von Wertetabellen gespeichert sind. Sprechen zwei Detektoren gleichzeitig an, kann aus derartigen Wertetabellen unmittelbar abgerufen werden, auf welchen Signallinien zwischen den beiden Detektoren das Ereignis mit welcher Wahrscheinlichkeit stattgefunden hat. Diese Wertetabellen sind gültig, solange die Geometrie des Scanners unverändert bleibt bzw. sich die Granularität des virtuellen Zylinders sich nicht ändert.

Der Scanner kann aber auch nach Alternative 1 Mittel zur Bestimmung der Wahrscheinlichkeitsverteilung enthalten. Unter einem Mittel zur Bestimmung der Wahrscheinlichkeitsverteilung ist beispielsweise ein Rechner zu verstehen, welcher die erfmdungsgemäßen Methoden anwendet. Diese Variante ist beispielsweise dann vorteilhaft, wenn der Scanner mechanisch so aufgebaut ist, dass er in seiner Geometrie veränderlich ist, beispielsweise, wenn er auf verschieden große Proben beziehungsweise Patienten einstellbar ist.

Erfindungsgemäß wird für die vom Detektor gelieferte Ortsinformation eine Likelihood-Verteilung $L\ (LORi\ |\ Kristallkombination\ j)$ ermittelt, aus der sich ein Verteilungsschlüssel bzw. Aufteilung der Information auf mehrere virtuelle TORs ableiten lässt. Dieser Verteilungsschlüssel kann vor jeder Messung einzeln oder einmal vor Inbetriebnahme des Scanners ermittelt und jeder weiteren Messung zu Grunde gelegt werden. Gegenstand der Erfindung ist daher ein Bildgebungsverfahren, welches von Projektionsdaten, die mit dem Verteilungsschlüssel generiert wurden, ausgeht sowie ein PET-Scanner, dessen gemessene Rohdaten mit dem Verteilungsschlüssel aufbereitet werden.

Spezielle Beschreibung:

Die Anwendung eines räumlich kontinuierlich variierenden Verteilungsschlüssels als Gegenstand dieser Erfindung verhindert die Inhomogenitäten im Projektionsraum grundsätzlich und resultiert in einer signifikanten Verbesserung des Kontrast-Rausch-Verhältnisses. Kernstück dabei ist die Generierung einer verbesserten scanner-spezifischen Likelihood-Verteilung $L(LOR\ i\ |\ Kristallkombination\ j),$ aus der sich unmittelbar ein solcher Verteilungsschlüssel ableiten lässt. Die Berücksichtigung der physikalischen Kristallgrößen kann vereinfacht durch ein geometrisches Monte Carlo Verfahren erfolgen. Dazu wird in den beiden Kristallen einer spezifischen 3D-Kristallkombination $j$ jeweils unabhängig ein Punkt gewürfelt, welche gemeinsam eine repräsentative 3D-LOR definieren, die jedenfalls zum LOR-Bündel der Kristallkombination gehört (Fig. 1 links). Diesem Ansatz liegt die Annahme zugrunde, dass ein Gamma-Quant mit jeweils homogener Wahrscheinlichkeit im gesamten Kristall eine nachweisbare Wechselwirkung verursachen kann. Somit lässt sich ein 3D-LOR-Bündel für jede beliebige Kristallkombination generieren, dessen statistische Präzision nur von der gewürfelten Punktdichteverteilung abhängt. Dabei treten alle beobachteten LORs in charakteristischer Häufung auf, da verschiedene Punktkombinationen jeweils die gleiche LOR i repräsentieren können (Fig. 1 Mitte). Diese Häufungsdichteverteilung der LORs i für eine Kristallkombination $j$ entspricht der gesuchten Likelihood-Verteilung $L(LOR\ i\ |\ Kristall\text{-}$

*kombination j).* Diese Verteilung kann genutzt werden, um die Messdaten in Sinograme/Histogramme über verschiedene Projektionsbins zu verteilen. Eine optimale Anwendung der Erfindung bietet das VRZ-Konzept, für welches alle generierten LOR auf einen regulären Zylinder in eindeutiger Weise projiziert werden. Die virtuelle Unterteilung auf dem Zylinder segmentiert den Projektionsraum zunächst in einzelne virtuelle 3D-Röhren (engl.: Tube-of-Response, kurz TOR), für welche die Rekonstruktion passende Projektoren bereitstellt. Jede virtuelle TOR definiert ein spezifisches eindeutiges 3D-LOR-Bündel und jede generierte LOR lässt sich genau einer solchen TOR, d. h. LOR-Bündel, zuordnen (Fig. 2). Daraus resultiert ein kompakter Verteilungsschlüssel, der den Beitrag einer Kristallkombination *j* zu jeder virtuellen TOR *k* festlegt. Sei $N_{tot,j}$ die Anzahl der generierten LORs für die Kristallkombination *j,* sowie *k* eine bestimmte TOR und $N_{jk}$ die Anzahl der generierten LORs, die im Bündel bzw. der TOR *k* liegen, so ergibt sich der normierte (geometrische) Verteilungsschlüssel zu

$$(2) \quad S_{jk} = N_{jk} / N_{tot,j} \; .$$

Dieser Scanner spezifische Verteilungsschlüssel unter Berücksichtigung der Detektorgeometrie wird vorab berechnet und jedes detektierte Koinzidenzereignis wird gemäß dieses Schlüssels auf die virtuellen TORs unter Erhaltung der Ereignisgesamtzahl aufgeteilt. Die effiziente Berechnung und Anwendung dieses Verteilungsschlüssels im Projektionsraum vor der eigentlichen iterativen Rekonstruktion ist Gegenstand dieser Erfindung.

Biespielhaft wird weiter ein Verfahren zur beschleunigten Berechnung des Verteilungsschlüssels auf Basis der Kristallgeometrien gemäß Gleichung (2) beschrieben. Die erzielte numerische Genauigkeit des obigen Monte Carlo Verfahrens hängt stark von der gewürfelten und ausgewerteten Anzahl an LORs für jede Kristallkombination ab, so dass eine befriedigende Genauigkeit erst für sehr hohe Punktdichten und damit verbundene sehr lange Rechenzeiten erreicht wird. Stattdessen lässt sich ein semi-analytisches Integralverfahren realisieren. Die Gewichtung mit der eine bestimmte LOR auftritt, ist aufgrund der Geometrie proportional zu dem Produkt aus den resultierenden Schnittlängen der jeweiligen LOR mit den beiden Kristallen (Fig. 1 rechts). Die beiden Schnittlängen $l_1$ und $l_2$ (3D Radon-Transformierten für eine bestimmte 3D-Signallinie der beiden Funktionen $f_{1,2}(x,y,z)=1$, falls $(x,y,z)$ in Kristall 1 bzw. 2 enthalten, sonst $f_{1,2}(x,y,z)=0$) lassen sich für jede beliebige LOR berechnen und das Produkt $p_{12}=l_1{}^*l_2$ ergibt das relative effektive Beitragsgewicht. Dadurch werden alle Punktkombinationen, die die betrachtete LOR erfasst, gleichzeitig berücksichtigt. Die Menge der zu betrachtenden LORs ist dabei wesentlich kleiner als beim obigen Monte Carlo-Verfahren bei gleicher Genauigkeit und verringert somit den Rechenaufwand. Zur Erhaltung der Gesamtereigniszahl werden die Verteilungsschlüssel für jede einzelne Kristallkombination *j* auf Eins normiert. Beide Verfahren ergeben im Rahmen der statistischen Genauigkeit in Abhängigkeit der Anzahl an ausgewerteten LORs äquivalente Verteilungsschlüssel, d. h. die Gewichte $S_{jk}$ konvergieren für hohe LOR-Abtastung zu den gleichen Werten.

Das primär vorgeschlagene Monte Carlo Verfahren bedarf mehr Rechenaufwand, bietet jedoch eine höhere Flexibilität in der Modellierung der Detektionswahrscheinlichkeiten, sofern diese nicht homogen in den beteiligten Kristallen ist. Ortsabhängige Detektionswahrscheinlichkeiten lassen sich durch die Einführung von gewichteten Punkten ebenfalls nachbilden. Dies stellt eine weitere Verbesserung der Likelihood-Modellierung der Ortsinformation dar. So können Punkte in der Nähe der Kristalloberflächen beispielsweise eine geringere Detektionswahrscheinlichkeit besitzen und durch entsprechend kleinere lokale Gewichte berücksichtigt werden.

Als weitere Eigenschaft der Erfindung lassen sich genaue Detektorsimulationen (z.B. mittels GEANT) für die Generierung einer Likelihood-Verteilung verwenden, welche dann sämtliche physikalische Effekte im Detetektionsprozess, z. B. Compton-Streuung in den Kristallen, richtig nachbildet. So lassen sich auch Verteilungsschlüssel mit einer sehr genauen Beschreibung des Systems einbinden und realisieren. Die Kombination von dezidierten Detektorsimulationen mit den Verteilungsschlüsseln sowie dem VRZ-Konzept eröffnen die Möglichkeit einer sehr genauen und trotzdem schnellen 3D-PET Rekonstruktion. Zwar sind GEANT - basierte Simulationen sehr langwierig, lassen sich jedoch einmalig durchführen und erlauben die Bestimmung eines sehr präzisen Verteilungsschlüssels. Dadurch hat die Dauer der Simulationen keinen weiteren Einfluss auf die benötigten Rekonstruktionszeiten. Bislang ist der Einsatz von genauen Detektorsimulationen nicht für eine routinemäßige Anwendung geeignet gewesen.

Darüber hinaus lassen sich durch die Anwendung eines Scanner spezifischen Verteilungsschlüssels allgemeine Rekonstruktionsprogramme (z. B. VRZ-Modell) implementieren und optimieren, die für verschiedene Scanner softwaretechnisch nicht mehr individuell angepasst werden müssen. Die Scanner-Geometrie und Datenkonvertierung ist ausschließlich im jeweiligen Verteilungsschlüssel enthalten, so dass gegenüber dem bisherigen Stand der Technik eine höhere Flexibilität und Portabilität für verschiedene Scanner-Systeme erreicht wird.

● Ausführungsbeispiel

Das erfindungsgemäße Verfahren kann beispielsweise auf dem Clear PET Neuro Small Animal PET Scanner [1] angewendet werden. Dieser Scanner besteht aus Detektorblöcken mit 8x8 Kristallen in zwei hintereinander liegenden Kristalllagen aus unterschiedlichen Kristallmaterialien. Die Kristallgröße beträgt 2 x 2 x 10 mm. Jeweils vier solcher Detektorblöcke sind axial angeordnet in einer so genannten Kassette. Das vollständige Detektorsystem ist ringförmig mit 20 dieser Kassetten bestückt, so dass insgesamt 10240 Kristalle existieren. Als Besonderheit rotiert das gesamte System axial um das Messobjekt während der Datenakquisition, um einen Teil der vorhandenen Lücken im Projektionsraum, resultierend aus Detektorlücken, auffüllen zu können. Die Rotation erfolgt in 1 Grad Winkelschritten, so dass in jeder einzelnen Detektorposition dann stationär Koinzidenzen gemessen werden.

Die mit den Clear PET Neuro System in 3D gemessenen PET Daten werden mit einem geometrischen Verteilungsschüssel, welcher die Kristallgrößen modelliert, in das VRZ-Modell mit gewählter hoher Granularität konvertiert. Für jede Detektorposition kommt der stationäre Verteilungsschlüssel gleichermaßen zur Anwendung. Die erhaltenen Teildaten lassen sich im Rahmen des VRZ-Modells anschließend verlustfrei entsprechend rotieren und werden dann zu einem Gesamtdatensatz aufsummiert. Der Verteilungsschlüssel wurde gemäß der beschriebenen semi-analytischen Integralmethode mit hoher Genauigkeit berechnet. Figur 3 zeigt exemplarisch ein Projektionsdatenhistogramm einer einzelnen Ringebene, welches mit dem Verteilungsschlüssel aus der Messung eines aktivitätsgefüllten homogenen Zylinderphantoms berechnet wurde. Die einzelnen rautenförmigen Datensegmente entsprechen den einzelnen virtuellen TORs (vgl. oben), welche jeweils in der Rekonstruktion mit einzelnen Projektoren verknüpft werden. Die Grauwerte entsprechen der zugehörigen Kanalintensität bzw. dem effektiven Projektionsmesswert. Die Sensitivitätslücken aufgrund von transaxialen Detektorlücken sind deutlich sichtbar. Mit zunehmender radialer Koordinate werden die geometrischen Strukturen aufgrund des zu erwartenden "Depth-of-Interaction"-Effekts verschmiert und entsprechen gemäß der zu Grunde liegenden Likelihood-Verteilung einer Verschlechterung der Auflösung, wie sie real im Scanner vorliegt.

Für das VRZ-Modell existiert eine iterative vollständige 3D Rekonstruktion. Die konvertierten Projektionsdaten lassen sich im Rahmen dieses Formalismus unmittelbar zu einem 3D-Bild rekonstruieren (Fig. 4). Das verwendete Mini-Derenzo-Phantom besteht aus KapillarStrukturen mit unterschiedlichen Kapillardurchmessern (2,0 mm, 1,8 mm, 1,6 mm, 1,4 mm, 1,2 mm, 1,0 mm) und wurde für die Messung axial ausgerichtet. Die Figur zeigt einen transaxialen Schnitt durch die Kapillaren. Die Kapillarstrukturen sind bis hinunter zu 1,2 mm sehr deutlich zu erkennen. Im Vergleich dazu zeigt Figur 5 eine Rekonstruktion der gleichen Messdaten basierend auf dem Stand der Technik durch die Verwendung von Sinogrammen mittels [2] ohne diese Erfindung. Der Unterschied im Kontrast-Rausch-Verhältnis ist offensichtlich. Die 1,2 mm Strukturen sind hier nicht mehr differenzierbar. Die Verwendung eines genauen Verteilungsschlüssels ergibt eine deutliche Verbesserung der Bildqualität.

[1] K. Ziemons et al., The Clear PET project: Development of a 2nd generation highperformance small animal PET scanner, Nucl. Instr. Meth A 537, 2005, pp.307-311

[2] C. Labbe, K. Thielemans, H. Zaidi, C. Morel, An object-oriented library incorporating efficient projection/backprojection operators for volume reconstruction in 3D PET, Proc. of 3D99 Conference, 1999, Egmond aan Zee, Netherlands

**Patentansprüche**

1. Verfahren zur Positronen-Emissions-Tomographie mit den Schritten:

   a) durch ein Annihilationsereignis in der Probe werden zwei Photonen in entgegen gesetzten Richtungen emittiert;
   b) von einer Vielzahl von um die Probe angeordneten Detektoren werden mindestens zwei durch die beiden Photonen zur Abgabe eines Signals veranlasst;
   c) aus dem Ort der Detektoren, die ein Signal abgegeben haben, wird eine Signallinie bestimmt, auf der das Ereignis stattgefunden haben kann;
   d) diese Signallinie wird bei der tomographischen Rekonstruktion der Probe ausgewertet,

   wobei zu jedem Ereignis eine Mehrzahl von Signallinien bestimmt und bei dieser tomographischen Rekonstruktion der Probe ausgewertet werden **dadurch gekennzeichnet, dass** jede Signallinie bei der Auswertung mit einer Wahrscheinlichkeitsverteilung gewichtet wird, die ein Maß dafür ist, auf welcher Signallinie das Ereignis mit welcher Wahrscheinlichkeit stattgefunden hat.

2. Verfahren nach Anspruch 1,
   **dadurch gekennzeichnet, dass** die Wahrscheinlichkeitsverteilung ermittelt wird, indem eine Vielzahl von innerhalb der beiden Detektoren gelegenen Orten zufällig bestimmt wird und Signallinien zwischen einem Ort in dem einen Detektor und einem Ort in dem anderen Detektor als Linien gewertet werden, auf denen das Ereignis stattgefunden haben kann.

3. Verfahren nach einem Anspruch 1 oder 2,
**dadurch gekennzeichnet, dass** die Wahrscheinlichkeitsverteilung ermittelt wird, indem für eine Vielzahl von Signallinien, die beide Detektoren schneiden, die Längen bestimmt werden, auf denen sie die Detektoren schneiden.

4. Verfahren nach Anspruch 3,
**dadurch gekennzeichnet, dass** das Produkt aus den Längen für eine Linie als Maß für die Wahrscheinlichkeit gewertet wird, dass das Ereignis auf dieser Linie stattgefunden hat.

5. Verfahren nach einem der Ansprüche 1 bis 4,
**dadurch gekennzeichnet, dass** bei der Auswertung die Ortsabhängigkeit der Sensitivität mindestens eines Detektors berücksichtigt wird.

6. Verfahren nach Anspruch 5 rückbezogen
**dadurch gekennzeichnet, dass** die Ortsabhängigkeit bereits bei der Ermittlung der Wahrscheinlichkeitsverteilung berücksichtigt wird.

7. Verfahren nach einem der Ansprüche 1 bis 6,
**dadurch gekennzeichnet, dass** vor der tomographischen Rekonstruktion die gewichteten Signallinien in ein oder mehrere Sinogramme überführt werden.

8. Verfahren nach einem der Ansprüche 1 bis 6,
**dadurch gekennzeichnet, dass** vor der tomographischen Rekonstruktion die Schnittpunkte jeder für diese Rekonstruktion in Betracht zu ziehenden Signallinien mit einer vorgegebenen geometrischen Form ermittelt und bei der Rekonstruktion so behandelt werden, als hätten sich dort Detektoren befunden.

9. Verfahren nach Anspruch 8,
**dadurch gekennzeichnet, dass** die geometrische Form für die Ermittlung der Schnittpunkte in eine endliche Anzahl von Intervallen diskretisiert wird.

10. Verfahren nach einem der Ansprüche 8 oder 9,
**dadurch gekennzeichnet, dass** ein Kreis oder ein Zylinder als geometrische Form gewählt wird.

11. Verfahren nach einem der Ansprüche 1 bis 10, **dadurch gekennzeichnet, dass** für die Generierung von Wahrscheinlichkeitsverteilungen Detektorsimulationen durchgeführt werden oder Messungen des Detektorverhaltens herangezogen werden.

12. Verfahren nach Anspruch 11,
**dadurch gekennzeichnet, dass** als Detektorsimulationen Monte Carlo-Verfahren eingesetzt werden.

**Claims**

1. Method for positron emission tomography, comprising the steps:

   a) two photons are emitted in opposing directions by an annihilation event in the sample;
   b) at least two of a large number of detectors arranged round the sample are prompted by the two photons to output a signal;
   c) a signal line on which the event can have occurred is determined from the location of the detectors which have output a signal;
   d) said signal line is evaluated in the tomographic reconstruction of the sample, wherein for each event, a plurality of signal lines is determined and evaluated in the tomographic reconstruction of the sample, **characterised in that**, during the evaluation, each signal line is weighted with a probability distribution which is a measure therefor on which signal line and with which probability the event has occurred.

2. Method according to claim 1, **characterised in that** the probability distribution is determined **in that** a large number of locations situated within the two detectors is randomly determined and signal lines between a location in one detector and a location in the other detector are evaluated as lines on which the event can have taken place.

3. Method according to claim 1 or 2, **characterised in that** the probability distribution is determined **in that**, for a large number of signal lines which intersect both detectors, the lengths at which said lines intersect the detectors are determined.

4. Method according to claim 3, **characterised in that** the product of the lengths for a line is evaluated as a measure for the probability that the event occurred on said line.

5. Method according to one of the claims 1 to 4, **characterised in that**, during evaluation of the location-dependency of the sensitivity, at least one detector is taken into account.

6. Method according to claim 5, **characterised in that** the location-dependency is already taken into account during determination of the probability distribution.

7. Method according to one of the claims 1 to 6, **characterised in that**, before the tomographic reconstruction, the weighted signal lines are converted into one or more sinograms.

8. Method according to one of the claims 1 to 6, **characterised in that**, before the tomographic reconstruction, the intersection points of each of the signal lines to be taken into account for said reconstruction with a pre-determined geometrical form are determined and, during the reconstruction, are treated as if the detectors were located there.

9. Method according to claim 8, **characterised in that** the geometric form for determining the intersection points is discretised into a finite number of intervals.

10. Method according to claim 8 or 9, **characterised in that** a circle or a cylinder is selected as the geometrical form.

11. Method according to one of the claims 1 to 10, **characterised in that**, for the generation of probability distributions, detector simulations are carried out or measurements of the detector behaviour are taken into account.

12. Method according to claim 11, **characterised in that** Monte Carlo methods are used as detector simulations.

**Revendications**

1. Procédé de tomographie par émission de positrons comportant les étapes suivantes :

   a) deux photons sont émis dans des directions opposées par un événement d'annihilation dans l'échantillon ;
   b) au moins deux parmi une pluralité de détecteurs disposés autour de l'échantillon sont amenés par les deux photons à émettre un signal ;
   c) est déterminée à partir de l'emplacement des détecteurs, qui ont émis un signal, une ligne de signal sur laquelle a pu avoir lieu l'événement ;
   d) ladite ligne de signal est évaluée lors de la reconstruction tomographique de l'échantillon,

   sachant qu'une pluralité de lignes de signal est déterminée pour chaque événement et est évaluée lors de ladite reconstruction tomographique de l'échantillon,
   **caractérisé en ce que** chaque ligne de signal est pondérée lors de l'évaluation grâce à une distribution de probabilité, laquelle constitue une mesure servant à déterminer la ligne de signal sur laquelle l'événement a eu lieu avec une telle probabilité.

2. Procédé selon la revendication 1,
   **caractérisé en ce que** la distribution de probabilité est déterminée **en ce qu'**une pluralité d'emplacements placés à l'intérieur des deux détecteurs est définie par hasard, et **en ce que** des lignes de signal entre un lieu dans l'un des détecteurs et un lieu dans l'autre détecteur sont considérées comme des lignes, sur lesquelles l'événement a pu avoir lieu.

3. Procédé selon l'une quelconque des revendications 1 ou 2,

caractérisé en ce que la distribution de probabilité est déterminée en ce que pour une pluralité de lignes de signal qui coupent les deux détecteurs, les longueurs sur lesquelles elles coupent les détecteurs sont définies.

4.  Procédé selon la revendication 3,
    caractérisé en ce que le produit des longueurs pour une ligne est considéré comme mesure pour la probabilité, en ce que l'événement a eu lieu sur ladite ligne.

5.  Procédé selon l'une quelconque des revendications 1 à 4,
    caractérisé en ce que lors de l'évaluation, on tient compte de la sensibilité variant en fonction du lieu d'au moins un détecteur.

6.  Procédé selon la revendication 5,
    caractérisé en ce qu'on tient compte des paramètres variant en fonction du lieu déjà lors de la détermination de la distribution de probabilité.

7.  Procédé selon l'une quelconque des revendications 1 à 6,
    caractérisé en ce que les lignes de signal pondérées sont transformées en un ou plusieurs sinogrammes avant la reconstruction tomographique.

8.  Procédé selon l'une quelconque des revendications 1 à 6,
    caractérisé en ce qu'on détermine avant la reconstruction tomographique les points de coupe de chacune des lignes de signal à considérer pour ladite reconstruction présentant une forme géométrique prédéfinie, et en ce qu'on les traite lors de la reconstruction comme si des détecteurs s'y étaient trouvés.

9.  Procédé selon la revendication 8,
    caractérisé en ce que la forme géométrique pour la détermination des points de coupe est discrétisée en un nombre infini d'intervalles.

10. Procédé selon l'une quelconque des revendications 8 ou 9,
    caractérisé en ce qu'on choisit comme forme géométrique un cercle ou un cylindre.

11. Procédé selon l'une quelconque des revendications 1 à 10, caractérisé en ce qu'on effectue pour générer des distributions de probabilité des simulations de détecteurs ou qu'on considère des mesures du comportement de détecteur.

12. Procédé selon la revendication 11,
    caractérisé en ce qu'on utilise en tant que simulations de détecteur des méthodes de Monte-Carlo.

Figur 1

Transaxial Ansicht (Modellbeispiel)

N Samplingpunkte
für jeden Kristall

Figur 2

Figur 3

Figur 4

Figur 5

Figur 6a                              Figur 6b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6804325 B1

**In der Beschreibung aufgeführte Nicht-Patentliteratur**

- **S. AGOSTINELLI et al.** GEANT-A simulation toolkit. *Nucl.Instr. Methods Phys. Res. A,* 2003, vol. 506, 250-303
- **K. ZIEMONS et al.** The Clear PET project: Development of a 2nd generation highperformance small animal PET scanner. *Nucl. Instr. Meth A,* 2005, vol. 537, 307-311
- **C. LABBE ; K. THIELEMANS ; H. ZAIDI ; C. MOREL.** An object-oriented library incorporating efficient projection/backprojection operators for volume reconstruction in 3D PET. *Proc. of 3D99 Conference,* 1999